(19) Europäisches Patentamt
European Patent Office
Office européen des brevets

(11) Publication number: **0 109 049 A2**

# (12) EUROPEAN PATENT APPLICATION

(21) Application number: **83111197.6**

(22) Date of filing: **09.11.83**

(51) Int. Cl.³: **C 07 D 211/90,** C 07 D 295/12, C 07 C 69/738 // A61K31/455, C07C79/46

(30) Priority: **15.11.82 US 441462**

(43) Date of publication of application: **23.05.84 Bulletin 84/21**

(84) Designated Contracting States: **AT BE CH DE FR GB IT LI LU NL SE**

(71) Applicant: **USV PHARMACEUTICAL CORPORATION, 1 Scarsdale Road, Tuckahoe New York (US)**

(72) Inventor: **Huang, Fu-chih, 611 Knoll Road, Boonton New Jersey (US)**
Inventor: **Lin, Clara J., 161 Moorland Drive, Scarsdale New York (US)**
Inventor: **Jones, Howard, 79 Briarcliff Woods, Ossining New York (US)**

(74) Representative: **Patentanwälte Grünecker, Dr. Kinkeldey, Dr. Stockmair, Dr. Schumann, Jakob, Dr. Bezold, Meister, Hilgers, Dr. Meyer-Plath, Maximilianstrasse 58, D-8000 München 22 (DE)**

(54) **Process for preparation of substituted dihydropyridines and intermediates therefor.**

(57) The invention provides, a new process for preparing dihydropyridines useful in the treatment of hypertension.

EP 0 109 049 A2

0102049
GRÜNECKER · DR. ... · DR. ...MAIR
DR. SCHUMANN · JAKOB · DR. BEZOLD · MEISTER
HILGERS · DR. MEYER · PLATH

# PROCESS FOR PREPARATION OF SUBSTITUTED DIHYDROPYRIDINES AND INTERMEDIATES THEREFOR

This invention relates to a new and useful process for preparing valuable, therapeutic dihydropyridines and intermediates useful in the preparation thereof.

In U.S. Patent 4,258,042, there is described a new class of substituted dihydropyridines which are particularly effective as anti-hypertensive agents. These compounds are N-morpholinoalkyl dihydropyridines of the formula:

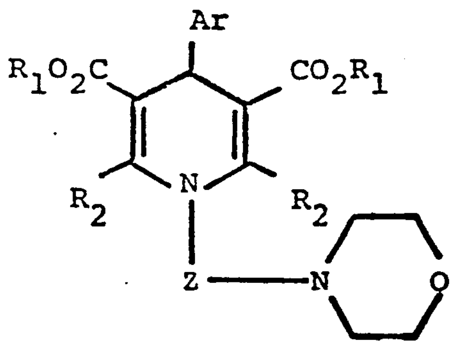

A

wherein Ar is heteroaryl, cycloalkyl having from 3 to 7 carbon atoms, naphthyl, indanyl, indenyl, tetrahydronaphthyl or a radical of formula:

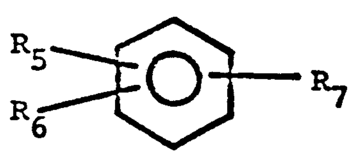

wherein each of $R_5$, $R_6$ and $R_7$ is independently H, alkyl, aryl, halo, lower alkoxy, nitro, amino, alkylmercapto, cyano, carboxy, carbalkoxy, sulfamyl, trifluoromethyl, hydroxy, acyloxy, methanesulfonyl, alkylamino or acylamino; and $R_5$ and $R_6$ when taken together form a methylenedioxy; Z is alkylene containing 1 to about 5 carbon atoms in the principal chain; and each $R_1$ is independently hydrogen, alkyl, or alkoxyalkyl, with the proviso that only one R may be hydrogen; each $R_2$ is independently lower alkyl; $R_3$ and $R_4$ are independently hydrogen or lower alkyl; and non-toxic, pharmaceutically acceptable acid addition salts thereof.

A procedure is described in the said patent for preparation of the aforesaid compounds in accordance with the following:

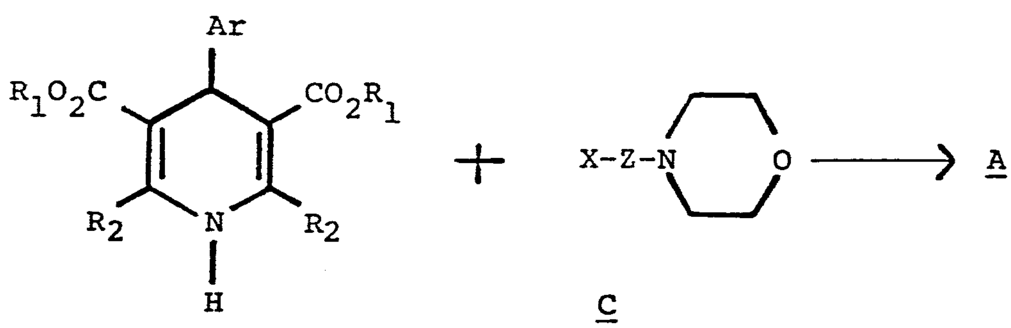

B C

Thus, a haloalkylmorpholine of formula C is reacted with a dihydropyridine of formula B, in the presence of sodium hydride for example and the desired product of formula A obtained. However, large excesses of sodium hydride are employed and this poses considerable difficulty in handling in commercial production. In addition, the

haloalkylmorpholines, e.g. N-(2-chloroethyl)morpholine, are not stable and cyclize on standing, necessitating storage in the form of the hydrochloride salt from which the free base must be produced by neutralization with base when required for reaction. Thus, the use of this procedure for commercial production of the desired therapeutic compounds is costly and time-consuming in view of the extra precautions and the base neutralization required.

Also described in the said patent is a condensation reaction of N-aminoalkylmorpholine, an arylaldehyde and acetoacetic acid ester; but the yields of this condensation reaction are quite low which renders this process economically unattractive for commercial production.

E. Wehinger et al. (The 182nd A.C.S. National Meeting, New York, N.Y. August 1981 Med. Chem. #64) described a process of synthesizing dihydropyridines of the formula:

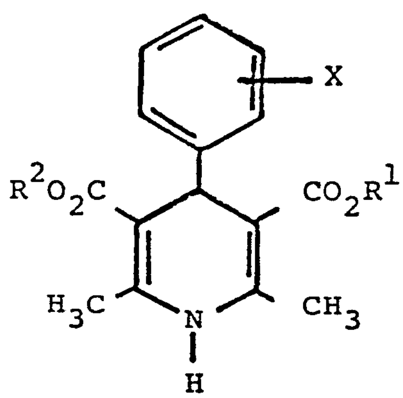

D

by reaction of a 2-benzylidene acetoacetate:

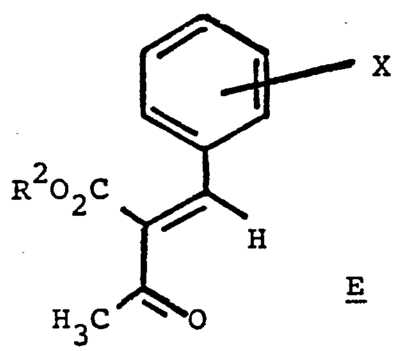

E

with an optically-active 3-aminocrotonic acid ester

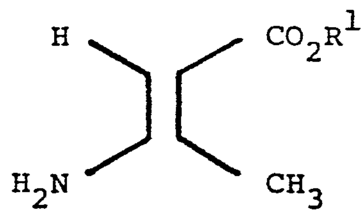

F

The present invention relates to the process of preparing 1,4-dihydro-4-aryl-2,6-disubstituted-1-substituted-3,5-pyridinedicarboxylate esters of the formula

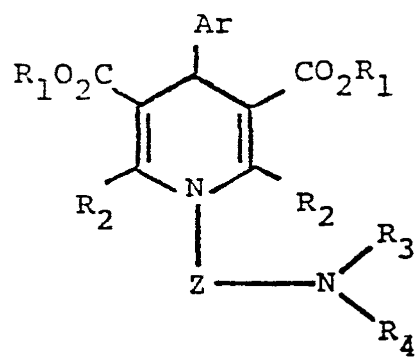

(I)

wherein Ar is heteroaryl, cycloalkyl having from 3 to 7 carbon atoms, naphthyl, indanyl, indenyl, tetrahydronaphthyl, or a radical of the formula

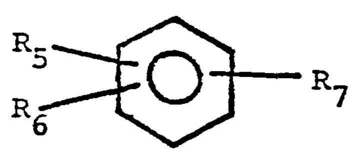

wherein each of $R_5$, $R_6$ and $R_7$ is independently H, alkyl, aryl, halo, lower alkoxy, nitro, amino, alkylmercapto, cyano, carboxy, carbalkoxy, sulfamyl, trifluoromethyl, hydroxy, acyloxy, methanesulfonyl, alkylamino or acylamino; and $R_5$ and $R_6$ when taken together, form a methylenedioxy; Z is alkylene containing 1 to about 5 carbon atoms in the principal chain; each $R_1$ is independently hydrogen, alkyl or alkoxyalkyl; each $R_2$ is independently lower alkyl; $R_3$ is hydroxyalkyl, aminoalkyl, alkoxyalkyl and $R_4$ is H, alkyl, cycloalkyl, hydroxyalkyl, aminoalkyl, alkoxyalkyl wherein the alkyl, alkoxy and acyl groups contain up to 10 carbon atoms and wherein $R_3$ and $R_4$ when taken together with the nitrogen to which they are attached form a heterocyclic ring which may be unsubstituted or substituted by hydrogen or alkyl selected from the group consisting of pyrrolyl, imidazolyl, pyrazolyl, dihydropyrazinyl, indolyl, isoindolyl, purinyl, perhydroquinolyl, perhydroisoquinolyl, carbaxolyl, carbolinyl (B), phenothiazinyl, phenoxozinyl, pyrrolidinyl, pyrrolinyl, pyrrolidonyl, pyrazolinyl pyrazolidinyl, imidazolinyl, imidazolidinyl, pyrazolyl, piperazinyl, o. and p. isoxazinyl, azepinyl, diazepinyl and morpholinyl which comprises condensing by water elimination a compound of the formula

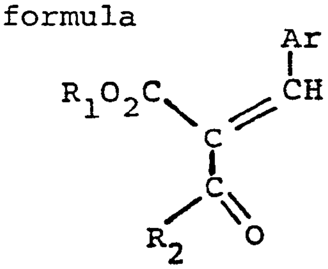

(II)

with a compound of the formula

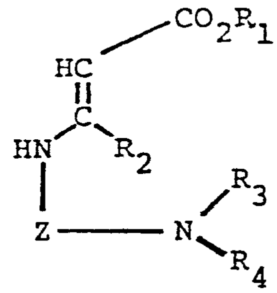

(III)

wherein Ar, Z, $R_1$, $R_2$, $R_3$, $R_4$, $R_5$, $R_6$ and $R_7$ are as hereinabove defined, and optionally forming the non-toxic pharmaceutically acceptable acid addition salts of the products (I).

The invention is particularly effective for the production of N-morpholinoalkyldihydropyridines of the formula:

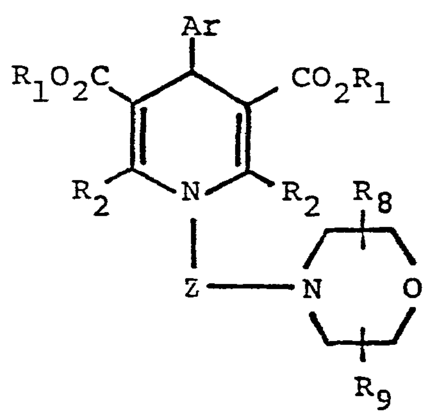

(IV)

0109049

wherein Ar, $R_1$, $R_2$ and Z are as previously described and $R_8$ and $R_9$ are each H or lower alkyl, which compounds are of particularly high efficacy in the treatment of hypertension.

The intermediate compounds of structure II are known compounds and preparable by art-recognized procedures.

The intermediate compounds of structure III however, are new compounds which are preparable by reacting an alkyl ester of a trans-3-aminocrotonic acid of the formula:

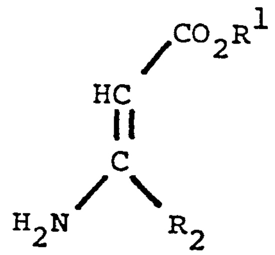

(V)

with an aminoalkyl substituted amine of the formula

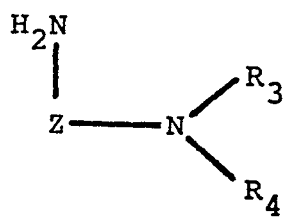

(VI)

usually in an inert organic solvent at temperatures of from about 80°C to about 200°C. The reactants can be heated without solvent present to obtain the desired product. The reaction time is usually from about 0.5 to about several hours depending on the selected reaction temperature, the higher the temperature the shorter the reaction time required for representative yields. The end-product of this reaction, i.e. the product of formula III, can be used without purification for the condensation with the formula II intermediates. Alternatively, if desired, the said end-product can be purified by standard methods prior to the condensation reaction.

The preferred compounds of structure III are those in which $-NR_3R_4$ is N-morpholino-, Z is ethyl, $R_8$ and $R_9$ are each H, $R_2$ is methyl and $R_1$ is ethyl or methyl. In addition, preferred compounds include those in which $R_3$ and $R_4$ are each hydroxyalkyl, especially hydroxyethyl; aminoalkyl, especially aminoethyl; or alkoxyalkyl, especially alkoxyethyl.

The condensation reaction between formula II and III intermediates to form the compounds of formula I is a dehydrating condensation, i.e. it involves splitting out water from the intermediates with the formation of the desired dihydropyridine ring characteristic of the formula I compounds but only when the trans-configuration of structure III compounds is used.

The dehydration can be effected using known dehydrating agents such as silica, preferably in the form of a sieve, e.g. molecular sieve, and is catalyzed by slightly acidic conditions. The slightly acidic condition can be effected employing amine hydrohalide salts, e.g. pyridine hydrochloride, diethylaniline hydrochloride, triethylamine hydrochloride, N-methyl piperidine hydrochloride, and similar amine sulfonate salts.

Dehydration can also be effected in the presence of reaction solvents which form azeotropes with water, such as toluene, and the water can be removed from the reaction mixture by refluxing and trapping the condensed water using, for example, a Dean Stark apparatus.

The dehydration may also be accomplished by absorption of water as formed by anhydrous salts which form thermally-stable hydrates.

The reaction mixture, including a solvent, is normally heated at elevated temperature for a time sufficient to accomplish significant dehydration. The time of reaction

of course is dependent on the selected reaction temperatures. Usually temperatures above about 100°C are employed, since the use of such temperatures permits reasonable reaction times which can range from 2 hours to 24 hours. It is normally convenient to conduct the reaction at the reflux temperature of the reaction mixture.

Solvents to be employed in the aforesaid reactions include, for example, tertiary amines, such as pyridine, N-methylpyrrolidine and N-methyl piperidine, aromatic hydrocarbon solvents such as benzene, toluene and xylene, tetrahydrofuran, dioxane, dimethylacetamide, acetic acid and lower alkanols such as ethanol and methanol. Of course, mixtures of solvents can be used.

The following examples further illustrate the invention.

EXAMPLE I

N-(2-morpholinoethyl)-4-(2-trifluoromethylphenyl)-2,6-dimethyl-3,5-dicarbethoxy-1,4-dihydropyridine

A. Ethyl 2-(o-trifluoromethylbenzylidene) Acetoacetic ester

A mixture of o-trifluoromethyl benzaldehyde (8.7 g), ethylacetoacetate (6.5 g), piperidine (0.2 ml), and acetic acid (0.6 ml) in 100 ml of toluene was heated to reflux while the water formed was removed by Dean-stark apparatus. Reaction was continued overnight. Evaporation of solvent gave 12 g of oily product. This was used for the next reaction without purification.

B. Ethyl 3-(N-morpholinoethylamino)crotonate

A mixture of ethyl trans-3-aminocrotonate (3 g) and N-(2-aminoethyl)morpholine (3 g) was heated at 140°C for 2 hours. The reaction product was used without further purification.

C.

N-(2-morpholinoethyl)-4-(2-trifluoromethylphenyl)-2,6-dimethyl-3,5-dicarbethoxy-1,4-dihydropyridine

A mixture of paragraph A product (247 mg); paragraph B product (242 mg), pyridine - hydrochloride (8 mg), and silica molecular sieve (3 g) in 20 ml of pyridine was heated at 140° for 19 hours. After evaporation of pyridine, the residue was extracted with ether. The organic solution was washed with water, 1 N sodium hydroxide solution, dried and concentrated. Purification by dry column gave 110 mg (20%) of product.

EXAMPLE II

(A) methyl 2-(o-nitrobenzylidene)-3-oxobutanoate

A solution of o-nitrobenzaldehyde (15.1 g, 0.10 mole 1, methyl acetoacetate (13.9 g, 0.12 mole), piperdine (0.2 ml) and acetic acid (0.6 ml) in toluene (100 ml) was heated to reflux with removal of water by means of a Dean-Stark strap overnight. The solvent was concentrated *in vacuo* and water was added to the residue. The product was extracted several times into methylene chloride, washed three times with water, dried over magnesium sulfate, filtered, and evaporated to give the product as an oil. The product was crystallized from n-hexane to afford pale yellow crystals, M.P. 73-76°.

(B) Ethyl 3-N-[2-(N-morpholino)ethyl]aminocrotonate

A mixture of methyl 3-aminocrotonate (3 g) and N-(2-aminoethyl)morpholine (3 g) was heated to 140° for four hours and then cooled to room temperature. The product was dissolved in methylene chloride, washed twice with water, dried over magnesium sulfate, filtered and evaporated to give the desired product as an oil which was used without further purification.

(C) Dimethyl 1,4-dihydro-2,6-dimethyl-1-[2-(4-morpholinyl) ethyl]-4-[2-(nitro)phenyl]-3,5-pyridinedicarboxylate

A mixture of methyl 2-(o-nitrobenzylidene)-3-oxobutoate (200 mg, 0.803 mmole), ethyl 3-N-[2-(N-morphalino)ethyl] aminocrotonate (200 mg, 0.877 mmole, pyridine hydrochloride (8 mg), pyridine (25 ml), and pulverized molecular sieves (3 g) was heated to reflux for six hours. The reaction was filtered and the filtrate was concentrated *in vacuo* to give a dark residue which was

chromatographed over silica-gel using chloroform/acetone as eluent. The desired fractions were combined and concentrated. The product crystallized as pale yellow crystals (125 mg, 34%) upon standing under ether/*n*-hexane, M.P. 161-163°.

Using the procedure of Example 2, paragraph B, the following crotonates are prepared from corresponding starting materials:

Ethyl 3-(N-methyl-N'-piperasinoethylamino)crotonate
Ethyl 3-[bis-(methyoxyethyl)aminoethylamino]crotonate
Ethyl 3-[bis-(hydroxyethyl)aminoethylamino]crotonate
Ethyl 3-(4-pyridine ethylamino)crotonate

and then reacted with the following acetoacetates to form corresponding dihydropyridines:

Ethyl 2-(o-nitrobenzylidene)acetoacetate
Ethyl 2-(2',3'-dichlorobenzylidene)acetoacetane
Ethyl 2-(o-trifluoromethylbenzylidene)acetoacetate

Using the foregoing crotonate and acetoacetate intermediates, the following dihydropyridines are produced:

N-(N-methylpiperazinoethyl)-4-(2-nitrophenyl)-2,6-dimethyl-3,5-dicarbethoxy-1,4-dihydropyridine
N-[bis-(hydroxyethyl)aminoethyl]-4-(2-trifluoromethylphenyl)-2,6-dimehtyl-3,5-dicarbethoxy-1,4-dihydropyridine
N-(4-pyridine-ethyl)-4-(2-nitrophenyl)-2,6-dimethyl-3,5-dicarbethoxy-1,4-dihyropyridine

WHAT IS CLAIMED IS:

1. The process of preparing 1,4-dihydro-4-aryl-2,6-disubstituted-1-(2-substituted)-3,5-pyridinedicarboxylate esters of the formula

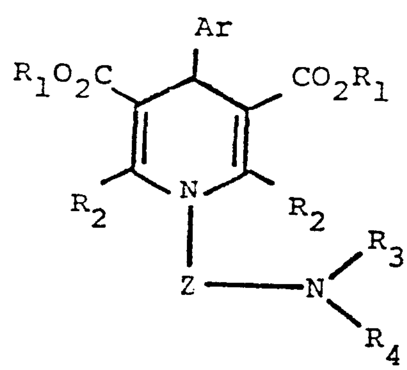

wherein Ar is heteroaryl, cycloalkyl having from 3 to 7 carbon atoms, naphthyl, indanyl, indenyl, tetrahydronaphthyl, or a radical of the formula

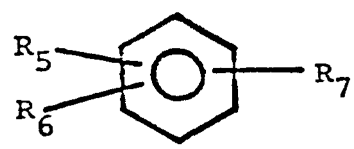

wherein each of $R_5$, $R_6$ and $R_7$ is independently H, alkyl, aryl, halo, lower alkoxy, nitro, amino, alkylmercapto, cyano, carboxy, carbalkoxy, sulfamyl, trifluoromethyl, hydroxy, acyloxy, methanesulfonyl, alkylamino or acylamino; and $R_5$ and $R_6$ when taken together, form a methylenedioxy; Z is alkylene containing 1 to about 5 carbon atoms in the principal chain; each $R_1$ is independently hydrogen, alkyl or alkoxyalkyl;

each $R_2$ is independently lower alkyl; $R_3$ is hydroxyalkyl, aminoalkyl, alkoxyalkyl and $R_4$ is H, alkyl, cycloalkyl, hydroxyalkyl, aminoalkyl, alkoxy- alkyl wherein the alkyl, alkoxy and acyl groups contain up to 10 carbon atoms and wherein $R_3$ and $R_4$ when taken together with the nitrogen to which they are attached form a heterocyclic ring which may be unsubstituted or substituted by hydrogen or alkyl selected from the group consisting of pyrrolyl, imidazolyl, pyrazolyl, dihydropyrazinyl, indolyl, isoindolyl, purinyl, perhydroquinolyl, perhydroisoquinolyl, carbazolyl, carbolinyl (B), phenothiazinyl, phenoxozinyl, pyrrolidinyl, pyrrolinyl, pyrrolidonyl, pyrazolinyl, pyrazolidinyl, imidazolinyl, imidazolidinyl, pyrazolyl, piperazinyl, o. and p. isoxazinyl, azepinyl, diazepinyl and morpholinyl which comprises condensing by water elimination a compound of the formula

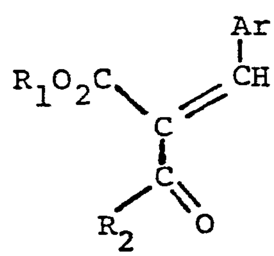

with a compound of the formula

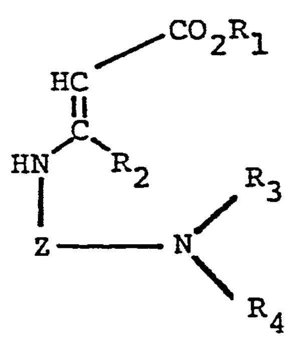

(III)

wherein Ar, Z, $R_1$, $R_2$, $R_3$, $R_4$, $R_5$, $R_6$ and $R_7$ are as hereinabove defined, and optionally forming the pharmaceutically acceptable acid addition salts of the products (I).

2. The process of preparing 1,4-dihydro-4-aryl-2,6-disubstituted-1-(2-morpholinoalkyl)-3,5-pyridinedicarboxylate esters of the formula

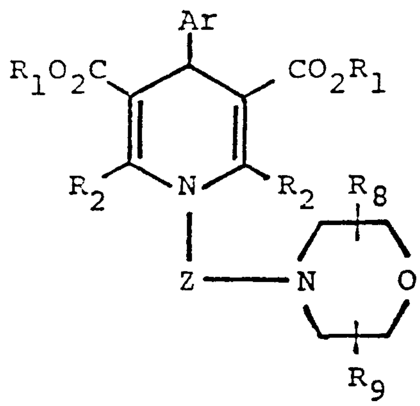

(I)

wherein Ar is heteroaryl, cycloalkyl having from 3 to 7 carbon atoms, naphthyl, indanyl, indenyl, tetrahydronaphthyl, or a radical of the formula

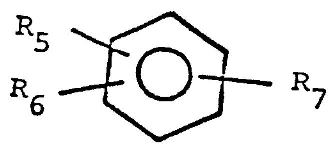

wherein each of $R_5$, $R_6$ and $R_7$ is independently H, alkyl, aryl, halo, lower alkoxy, nitro, amino, alkylmercapto, cyano, carboxy, carbalkoxy, sulfamyl, trifluoromethyl, hydroxy, acyloxy, methanesulfonyl, alkylamino or acylamino; and $R_5$ and $R_6$, when taken together, form a methylenedioxy; Z is alkylene containing 1 to about 5 carbon atoms in the principal chain; each $R_1$ is independently hydrogen, alkyl or alkoxyalkyl, with the proviso that only one $R_1$ may be hydrogen; each $R_2$ is independently lower alkyl; and $R_8$ and $R_9$ are each H or lower alkyl; which comprises condensing by water elimination a 2-arylidine acetoacetic ester of the formula

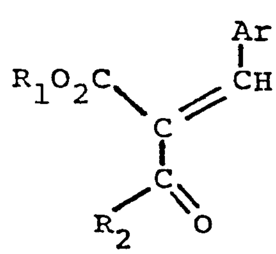

with a 3-aminocrotonic acid ester of the formula

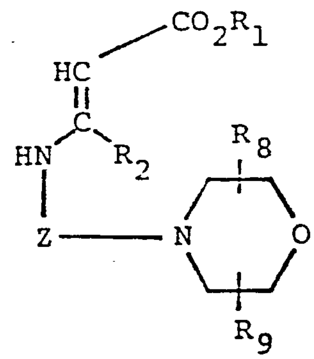

wherein Ar, Z, $R_1$, $R_2$, $R_5$, $R_6$, $R_7$, $R_8$ and $R_9$ are as hereinabove defined, and optionally forming the pharmaceutically acceptable acid addition salts of the products.

3. The process of Claim 2 wherein the condensation is conducted in an inert organic solvent under reflux conditions.

4. The process of Claim 2 wherein the condensation is conducted in the presence of pyridine hydrochloride and molecular sieve in an inert organic solvent at a temperature of from about 80°C. to about 200°C.

5. The process of Claim 3 wherein said solvent forms an azeotrope with water.

6. The process of Claim 2 wherein Ar is o-trifluoromethylphenyl, $R_1$ is ethyl, $R_2$ is methyl, Z is ethylene and $R_8$ and $R_9$ are each hydrogen.

7. The process of Claim 2 wherein Ar is o-nitrophenyl, $R_1$ is ethyl, $R_2$ is methyl, Z is ethylene and $R_8$ and $R_9$ are each hydrogen.

8. A compound of the formula

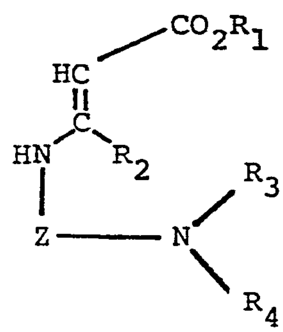

wherein Z is alkylene containing 1 to about 5 carbon atoms in the principal chain; $R_1$ is hydrogen, alkyl or alkoxyalkyl; $R_2$ is lower alkyl; $R_3$ is hydroxyalkyl, aminoalkyl or alkoxyalkyl and $R_4$ is H, alkyl, cycloalkyl, hydroxyalkyl, aminoalkyl, alkoxyalkyl wherein the alkyl and alkoxy groups contain up to 10 carbon atoms and wherein $R_3$ and $R_4$ when taken together with the nitrogen to which they are attached form a heterocyclic ring which may be substituted by hydrogen or alkyl selected from the group consisting of pyrrolyl, imidazolyl, pyrazolyl, dihydropyrazinyl, indolyl, isoindolyl, purinyl, perhydro- quinolyl, perhydroisoquinolyl, carbazolyl, carbolinyl, phenothiazinyl, phenoxozinyl, pyrrolidinyl, pyrrolinyl, pyrrolindonyl, pyrazolinyl, pyrazolidinyl, imidazolinyl, imidazolidinyl, pyrazolyl, piperazinyl, o. and p. isoxazinyl, azepinyl, diazepinyl and morpholinyl.

9. A compound according to Claim 8 wherein $R_1$ is ethyl, $R_2$ is methyl, Z is ethylene and $R_3$ and $R_4$ are each hydroxyethyl.

10. A compound according to Claim 8 wherein $R_3$ and $R_4$ are each hydroxyalkyl.

11. A compound according to Claim 8 wherein $R_3$ and $R_4$ are each hydroxyethyl.

12. A compound according to Claim 8 wherein $R_3$ and $R_4$ are each aminoalkyl.

13. A compound according to Claim 8 wherein $R_3$ and $R_4$ are each aminoethyl.

14. A compound according to Claim 8 wherein $R_3$ and $R_4$ are each alkoxyalkyl.

15. A compound according to Claim 8 wherein $R_3$ and $R_4$ are each alkoxyethyl.

16. Compounds having the formula

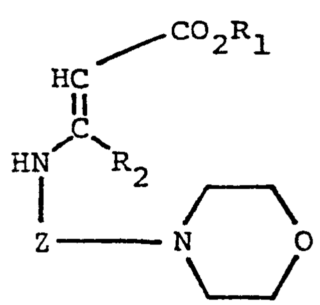

wherein $R_1$ is hydrogen, alkyl or alkoxyalkyl, $R_2$ is lower alkyl, and Z is alkylene containing 1 to about 5 carbon atoms.

17. The compound of Claim 16 wherein $R_1$ is ethyl, $R_2$ is methyl and Z is ethylene.